# EUROPEAN PATENT APPLICATION

(11) **EP 3 915 562 A1**
(43) Date of publication of application: **01.12.2021**
(21) Application number: 21157459.5
(22) Date of filing: 16.02.2021
(51) Int. Cl.: A61K 31/4706, A61P 17/00, A61P 29/00, A61P 37/00, A61K 9/00

(54) **TREATMENT OF LUPUS ERYTHEMATOSUS USING S- HYDROXYCHLOROQUINE**

(30) Priority: 31.05.2020 US 202063032658 P
(71) Applicant: Genovate Biotechnology Co., Ltd., Hsin-Chu Expanded Industrial Park Hsin-Chu 300 (TW)
(72) Inventor: Chen, Jen, Hsin-Chu (TW); Yao, Nai-tung, Hsin-Chu (TW)
(74) Representative: Cabinet Becker et Associés

(57) **Abstract**

The invention provides a pharmaceutical composition that contains S-hydroxychloroquine (S-HCQ) and a pharmaceutically acceptable excipient, for use in treating lupus erythematosus. The pharmaceutical composition is essentially free of R-hydroxychloroquine (R-HCQ).

## Description

### Background

Hydroxychloroquine ("HCQ") is included in the World Health Organization Model List of essential medicines for treating malaria caused by *Plasmodium vivax.* This drug is also an important treatment for chronic polymorphous solar eruption, as well as for rheumatologic diseases such as cutaneous (i.e., discoid) and systemic lupus erythematosus and rheumatoid arthritis.

HCQ has two optical isomers, i.e., the (R)-(-)-isomer ("R-HCQ") and the (S)-(+) isomer ("S-HCQ"). For treating the above conditions, it is administered as a racemic (50:50) mixture of these two isomers.

Long-term and high-dose administration of HCQ can cause blurred vision and in some cases can damage the retina, cornea, or macula and lead to vision impairments in some patients due to its accumulation in ocular tissues.

HCQ is also known to be cardiotoxic. It can cause interventricular conduction delay (i.e., QRS widening), Q wave to T wave (QT) interval prolongation (i.e., long QT syndrome), Torsades de pointes, ventricular arrhythmia, hypokalemia, and hypotension

It has been reported that some patients being treated for lupus erythematosus with long-term HCQ therapy developed acquired long QT syndrome with refractory ventricular arrhythmia, which can result in sudden death.

Long-term administration of HCQ for treating lupus erythematosus should be balanced against the risk of developing potentially lethal cardiac arrhythmias.

Methods are needed for effectively treating cutaneous and systemic lupus erythematosus without the side-effects mentioned above.

### Summary

To meet the above need, it is herein provided a pharmaceutical composition that contains S-hydroxychloroquine (S-HCQ) and a pharmaceutically acceptable excipient, for use in treating lupus erythematosus. The pharmaceutical composition is essentially free of R-hydroxychloroquine (R-HCQ).

In this context, a pharmaceutical composition containing S-HCQ that is "essentially free" of R-HCQ includes S-HCQ in an enantiomeric excess over R-HCQ of not less than 99%. As such, a pharmaceutical composition essentially free of R-HCQ includes compositions in which the enantiomeric excess of S-HCQ is 99%, 99.1%, 99.2%, 99.3%, 99.4%, 99.5%, 99.6%, 99.7%, 99.8%, 99.9% and 100%.

The method is effective for treating both systemic lupus erythematosus and cutaneous lupus erythematosus. Patients suffering from systemic lupus erythematosus that have concurrent lupus nephritis can also be treated according to the present invention.

Administration of S-HCQ according to the present invention has fewer side effects, particularly respecting cardiotoxicity, as compared to R-HCQ and to an equimolar mixture of S-HCQ and R-HCQ, i.e., HCQ.

The details of several embodiments of the present invention are set forth in both the description and the drawings below.

### Brief Description of the Drawings

The description below refers to the accompanying drawings, of which:
Fig. 1A is a plot of body weight versus time for vehicle treated MRL/lpr mice or MRL/lpr mice treated with a 20 mg/kg daily dose of hydroxychloroquine (GVX 170), S-hydroxychloroquine (GVX 171), or R-hydroxychloroquine (GVX 172). n=15 for vehicle treated and n=14 for each treatment.
Fig. 1B is a plot of body weight versus time for vehicle treated MRL/lpr mice or MRL/lpr mice treated with a 40 mg/kg daily dose of hydroxychloroquine, S-hydroxychloroquine, or R-hydroxychloroquine. Legend and number of mice per treatment are as in Fig. 1A.
Fig. 2A is a Kaplan-Meier survival plot of MRL/lpr mice treated daily with vehicle or with 20 mg/kg of the indicated compounds. Legend and number of mice per treatment are as in Fig. 1A.
Fig. 2B is a Kaplan-Meier survival plot of MRL/lpr mice treated daily with vehicle or with 40 mg/kg of the indicated compounds. Legend and number of mice per treatment are as in Fig. 1A.
Fig. 3A is a plot of mean skin lesion score versus days from start of treatment for MRL/lpr mice treated daily with vehicle, or with 20 mg/kg or 40 mg/kg of the indicated compounds. Legend and number of mice per treatment are as in Fig. 1A.
Fig. 3B is a bar graph showing mean skin lesion score over days 68-111 for the indicated control and 20 mg/kg treatment groups. Legend and number of mice per treatment are as in Fig. 1A. ** = significantly different from vehicle group at p < 0.005 by t-test.
Fig. 3C is a bar graph showing mean skin lesion score over days 68-90 for the indicated control and 40 mg/kg treatment groups. Legend and number of mice per treatment are as in Fig. 1A. ** = significantly different from vehicle group at p < 0.005 by t-test.
Fig. 4A is a bar graph showing blood urea nitrogen (BUN) levels in plasma of MRL/lpr mice treated with vehicle or with the indicated drugs/dosages. Legend and number of mice per treatment are as in Fig. 1A. * = significantly different from vehicle group at p < 0.05 by t-test.
Fig. 4B is a bar graph showing creatinine levels in plasma of MRL/lpr mice treated with vehicle or with the indicated drugs/dosages. Legend and number of mice per treatment are as in Fig. 1A. * = significantly different from vehicle group at p < 0.05 by t-test; ** = significantly different from vehicle group at p < 0.01 by t-test.

### Detailed Description

It is herein described a method is provided for treating lupus erythematosus by administering to an affected subject a pharmaceutical composition that contains S-HCQ and a pharmaceutically acceptable excipient in which the pharmaceutical composition is essentially free of R-HCQ.

The pharmaceutical composition containing S-HCQ and being essentially free of R-HCQ includes S-HCQ in an enantiomeric excess of not less than 99%, defined as the percentage of the total amount of S-HCQ and R-HCQ in the composition that is S-HCQ minus the percentage that is R-HCQ. For example, a pharmaceutical composition of the invention having S-HCQ in an enantiomeric excess of 99% contains 99.5% by weight S-HCQ and 0.5% by weight R-HCQ, based on the total weight of S-HCQ plus R-HCQ in the composition. The pharmaceutical composition essentially free of R-HCQ can have an enantiomeric excess of S-HCQ of 99%, 99.1%, 99.2%, 99.3%, 99.4%, 99.5%, 99.6%, 99.7%, 99.8%, 99.9% and 100%.

S-HCQ preparations having the above enantiomeric excess can be produced by the methods set forth in US Provisional Patent Application Serial No. 63/013,219 and US

Patent 5,314,894.

The S-HCQ in the pharmaceutical composition can be in free base form or in the form of a pharmaceutically acceptable salt. The pharmaceutically acceptable salt can be, but is not limited to, a sulfate salt, a phosphate salt, and a hydrochloride salt. In a particular example of the pharmaceutical composition, the S-HCQ is a sulfate salt.

To carry out the treatment method, the subject suffering from lupus erythematosus is administered an amount of the pharmaceutical composition corresponding to a daily dose of 100 mg to 800 mg (e.g., 100, 200, 300, 400, 500, 600, 700, and 800 mg) of S-HCQ. In a specific method, 200 mg per day of S-HCQ is administered.

In the above method, the pharmaceutical composition can be in the form of granules, a tablet, a capsule, a pill, a powder, a solution, a suspension, or a syrup. An exemplary pharmaceutical composition is in the form of a pill for oral administration.

Preparation of the granules, tablet, capsule, pill, powder, solution, suspension, and syrup can be by conventional techniques, such as those described in US Patent Application Publication 2018/0194719.

As mentioned above, the pharmaceutical composition includes a pharmaceutically acceptable excipient. A pharmaceutically acceptable excipient can be any physiologically inert excipient used in the pharmaceutical art of dispensing, including but not limited to binders, diluents, surfactants, disintegrants, lubricants/glidants, and coloring agents. Particular excipients can be those described in US Patent Application Publication 2008/020634.

The above-described method can be used for treating a subject suffering from systemic lupus erythematosus or cutaneous lupus erythematosus. Treatment of an individual having systemic lupus erythematosus that has concurrent lupus nephritis is also within the scope of the invention.

Without further elaboration, it is believed that one skilled in the art can, based on the disclosure herein, utilize the present disclosure to its fullest extent. The following specific examples are, therefore, to be construed as merely descriptive, and not limitative of the remainder of the disclosure in any way whatsoever.

### EXAMPLES

### Example 1: Effect of HCQ, S-HCQ, and R-HCQ on the synthesis of IL-17 by Phorbol Myristate Acetate/Ionomycin-Stimulated PBMC

Hydroxychloroquine (HCQ) and its pure optical isomers S-hydroxychloroquine (S-HCQ; enantiomeric excess of 99.0%) and R-hydroxychloroquine (R-HCQ; enantiomeric excess of 98.3%) were tested in an *in vitro* pharmacodynamic assay to determine their efficacy at inhibiting interleukin 17 (IL-17) production in human peripheral blood mononuclear cells (PBMC) stimulated with phorbol myristate acetate (PMA) and ionomycin.

Human PBMC were purchased from ATCC® (PCS-800-011™) and thawed as suggested by the provider. Cells were cultured at 37 °C with 5% CO₂ in RPMI-1640 medium (Gibco, 11875-093) supplemented with 10% Fetal Bovine Serum (Gibco, 10437-028), 10 mM HEPES (Gibco, 15630-080), and 100 U/mL Penicillin/Streptomycin (Gibco, 15140-122).

PBMC were seeded at a density of 10⁶ cells/ml in each well of a 96-well round bottom microplate (Corning, 7007) then stimulated with 25 ng/ml PMA and 1 µg/ml ionomycin in the absence and presence of 25 µM, 50 µM, or 100 µM HCQ, S-HCQ or R-HCQ each dissolved in ethanol. The final concentration of ethanol in the culture medium was 0.75%. PBMC treated with 5 µM methylprednisolone served as a positive control.

After incubation for 48 or 72 hours, the cell culture supernatants were collected and stored at -80 °C until analysis. The levels of IL-17 induced by PMA/ionomycin-stimulated PBMC were analyzed using human IL-17 Quantikine ELISA Kit (R & D system, D1700) according to product information. The results are shown in Table 1 below.

The levels of IL-17 produced by PMA/ionomycin-stimulated PBMC were inhibited in a dose dependent manner when incubated for 48 h or 72 h in the presence of each of HCQ, S-HCQ, and R-HCQ. The absolute IC₅₀ of IL-17 inhibition by HCQ, S-HCQ or R-HCQ was between 50-100 µM. There were no major differences, i.e., at least 20 percentage points, between the inhibition rate of HCQ, S-HCQ and R- HCQ on IL-17 production induced by PMA/ionomycin in PBMC.

**Table 1. Effect on synthesis of IL-17 in PMA/Ionomycin-stimulated PBMC**

| | | 48 h | | 72 h | |
|---|---|---|---|---|---|
| Group (n = 3/group) | | IL-17 pg/mL (mean ± SD) | (%) Inhibition | IL-17 pg/mL (mean ± SD) | (%)Inhibition |
| Blank Control | | ND^{a} | 100 | ND | 100 |
| PMA/ionomycin Control | | 494.5 ± 2.3 | 0 | 649.4 ± 113.3 | 0 |
| 5 µM methylprednisolone | | 266.7 ± 15.3 | 46 | 282.0 ± 37.6 | 57 |
| hydroxychloroquine | 25 µM | 480.4 ± 13.1 | 3 | 502.4 ± 20.7 | 23 |
| | 50 µM | 344.8 ± 12.2 | 30 | 341.1 ± 47.4 | 48 |
| | 100 µM | 214.5 ± 15.3 | 57 | 203.0 ± 10.8 | 69 |
| S-hydroxychloroquine | 25 µM | 432.6 ± 28.9 | 13 | 486.5 ± 8.9 | 25 |
| | 50 µM | 292.0 ± 21.6 | 41 | 354.6 ± 52.5 | 45 |
| | 100 µM | 215.0 ± 24.6 | 57 | 248.9 ± 38.9 | 62 |
| R-hydroxychloroquine | 25 µM | 471.6 ± 32.9 | 5 | 564.0 ± 72.9 | 13 |
| | 50 µM | 289.4 ± 34.7 | 42 | 326.8 ± 33.6 | 50 |
| | 100 µM | 154.0 ± 18.1 | 69 | 179.2 ± 31.8 | 72 |

| | | | | | |
|---|---|---|---|---|---|
| ^{a} ND = not detectable | | | | | |

### Example 2: Pharmacodynamics

Pharmacodynamics of HCQ, S-HCQ, and R-HCQ was examined in MRL/lpr mice, a model for human cutaneous lupus erythematosus. See Furukawa et al., 1996, J. Invest. Dermatol. 107;95-100; Furukawa et al., 2009, Autoimmun. Rev. 8:495-499; and Shimomatsu et al., 2016, Mod. Rheumatol. 26:744-748. Eight-week old MRL/lpr mice, were purchased from Jackson Laboratory (ME, USA) and maintained on a 12 h light and dark cycle with constant temperature and humidity control. Animals were given water and food *ad-libitum.* The S-HCQ and R-HCQ used had the enantiomeric excesses set forth above in Example 1.

Groups of 14-15 mice were treated starting at the age of ∼15 weeks old with vehicle (10% EtOH in PBS), with low dose (20 mg/kg) or high dose (40 mg/kg) HCQ, S-HCQ, or R-HCQ. Mice in the vehicle and low dose groups were treated orally once daily for 16 weeks from age 15 to 31 weeks. Mice in the high dose groups were treated orally once daily for 14 weeks from age 15 to 29 weeks. Dosing volumes were kept consistent at 10 mL/kg.

### Body weight of treated MRL/lpr mice

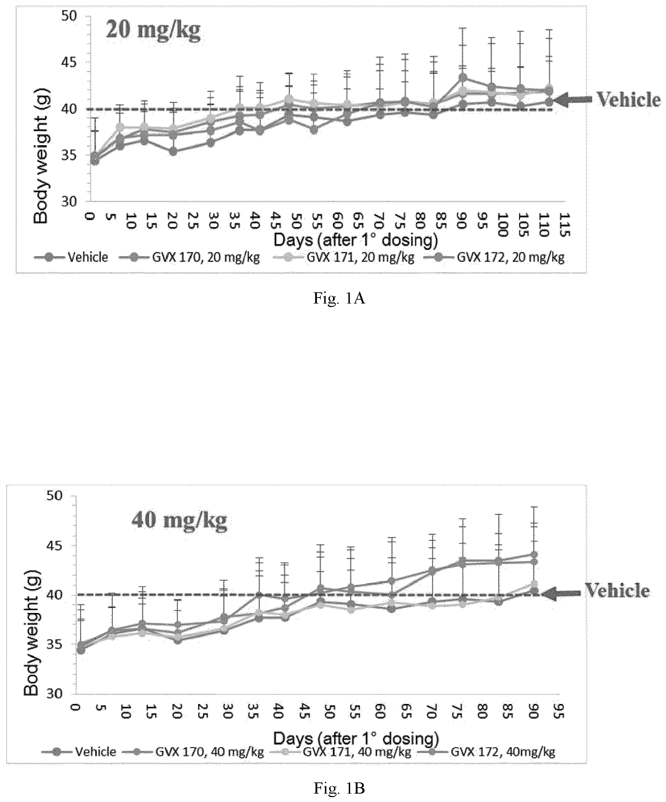

MRL/lpr mice treated with vehicle or with 20 mg/kg HCQ, S-HCQ, or R-HCQ were weighed once per week from day 0 (initial day of dosing) to day 111. Animals in the 40 mg/kg groups were weighed daily from day 0 to day 90. No obvious difference in mean body weight was observed between vehicle and all 20 mg/kg groups (see Fig. 1A) and between vehicle and all 40 mg/kg groups (see Fig. 1B).

### Survival of treated MRL/lpr mice

The number of live and dead mice was recorded each day beginning from the first dosing day at age 15 weeks. Survival was observed for 10-11 weeks (70-80 days) after dosing began.

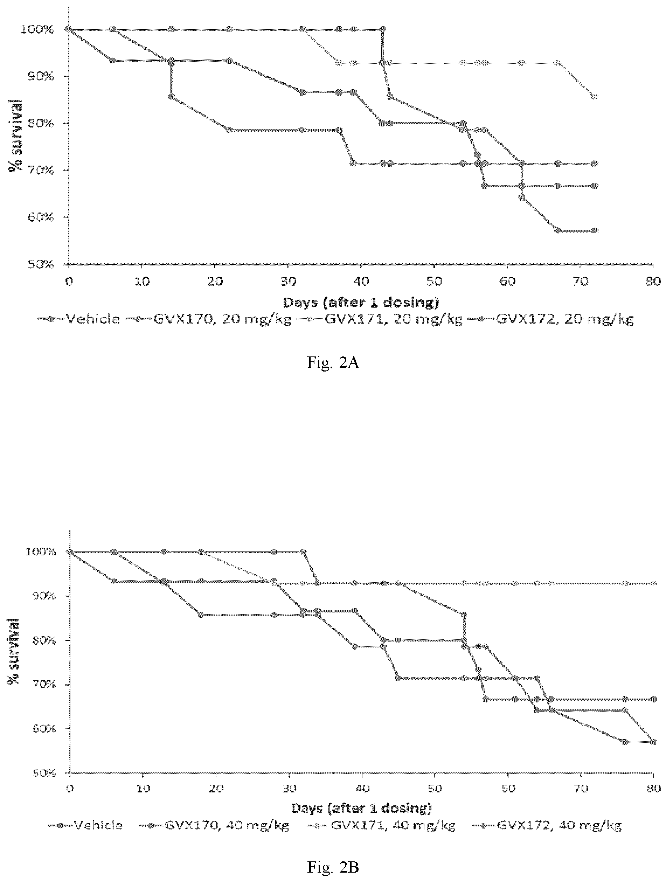

The results are shown in Figs. 2A and 2B. Throughout the observation period, mice treated with S-HCQ showed a higher fractional survival compared to mice treated with vehicle, HCQ and R-HCQ in both the 20 mg/kg (Fig. 2A) and 40 mg/kg (Fig. 2B) dosing groups.

### Skin lesions in treated MRL/lpr mice

Inflammation and skin lesions in the MRL/lpr mice were scored twice per week from the regions of dorsal neck, forehead, snout, and ears using a previously described scoring scale. See Keil et al., 2016, Arthritis Res. Ther. 18:243. Briefly, animals with no skin lesions were scored as 0, those having minimal hair loss with redness and a few scattered lesions were scored as 1, and those with redness, scabbing, and hair loss with a small area of involvement were scored as 2.

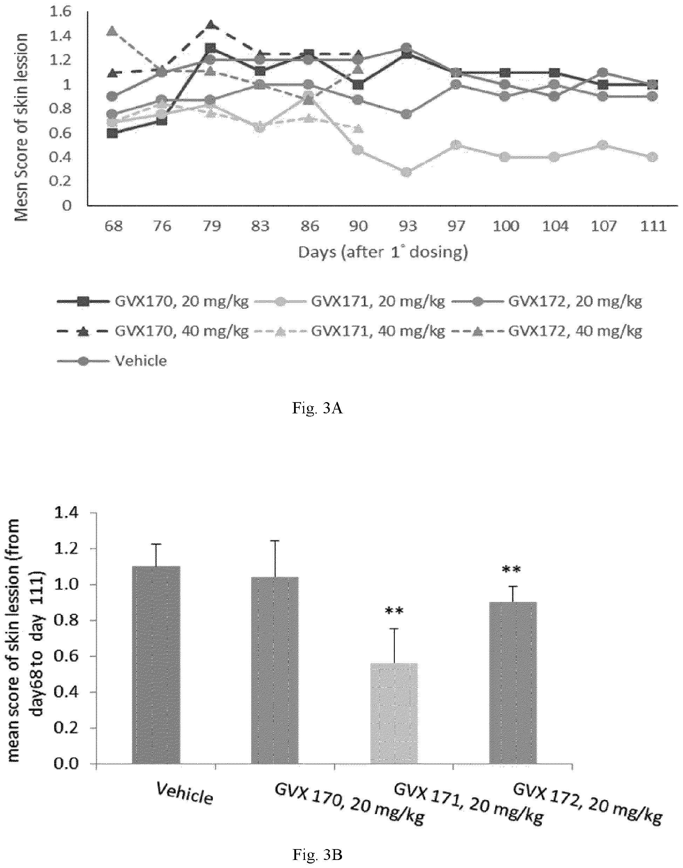

The mean score of skin lesions in each group from Day 68 to Day 111 for vehicle and low dose groups and from Day 68 to Day 90 for high dose groups after first dosing were plotted and are shown in Fig. 3A. The data shows that treatment with S-HCQ at both 20 mg/kg and 40 mg/kg was superior in reducing the onset of skin inflammation and lesions in the MRL/lpr mice, as compared to vehicle and to HCQ and R-HCQ treatments.

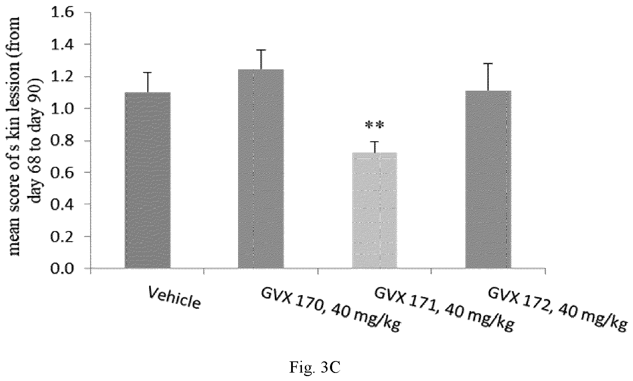

The data was reanalyzed by comparing the average skin lesion scores for each treatment over the entire test period. The skin lesion scores from day 68 to day 111 after initial dosing were averaged for the vehicle and the low dose groups and compared, as shown in Fig. 3B. The skin lesion scores from day 68 to day 90 after initial dosing were averaged for the vehicle and the high dose groups and compared, as shown in Fig. 3C. Only S-HCQ significantly reduced skin lesion scores over the test period at both low and high doses.

The results indicated that S-HCQ was superior to other tested treatments for reducing the spontaneous development of lupus-like skin lesions in MRL/lpr mice.

### Blood urea nitrogen and creatinine concentration

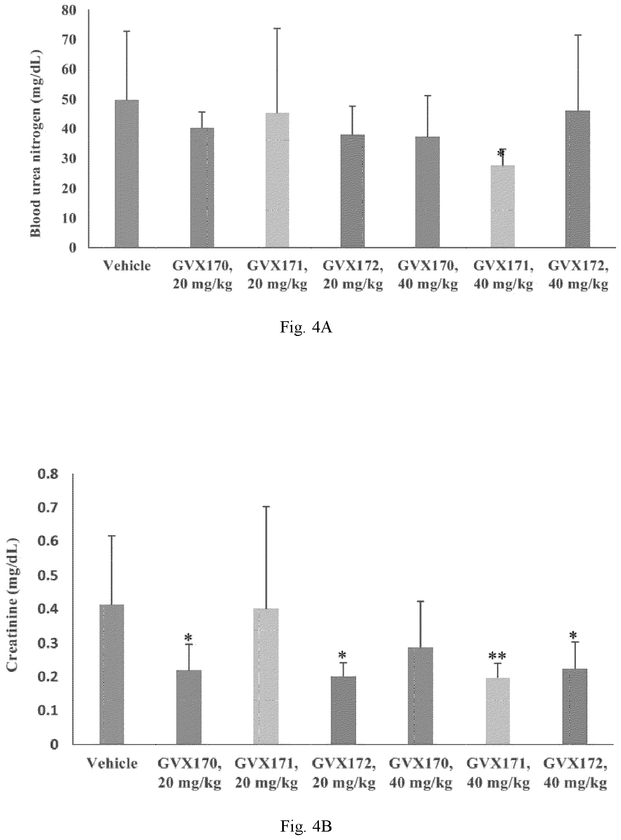

Plasma was prepared from blood samples collected at the end of the study. They were analyzed for blood urea nitrogen (BUN) level and creatinine concentration using a FUJI DRI-CHEM 4000i instrument as directed by the manufacturer. Elevated BUN and creatinine levels indicate compromised kidney function. The results are shown in Figs. 4A and 4B.

BUN levels were significantly lower in MRL/lpr mice treated with 40 mg/kg of S-HCQ compared to those animals subjected to any other treatments. See Fig. 4A.

Turning to creatinine concentration, it was lower in mice treated with HCQ (20 mg/kg) and R-HCQ (20 mg/kg and 40 mg/kg). See Fig. 4B. The most significant decrease in creatinine concentration was seen in animals treated with 40 mg/kg S-HCQ. See *Id.*

### Example 3: In vitro cardiotoxicity assay

The predicted cardiac toxicity of HCQ, S-HCQ, and R-HCQ was examined by determining the ability of these drugs to inhibit the activity of the human ether-a-go-go-related gene (hERG) voltage-gated potassium channel. Inhibition of hERG *in vivo* by a drug can result in acquired long QT syndrome, which often leads to torsade de pointes cardiac arrythmia and sudden death. S-HCQ and R-HCQ used in this study had an enantiomeric excess of 99.8% and 98.3%, respectively.

A conventional patch-clamp technique was used to determine inhibitory effects on hERG. Human embryonic kidney cells (HEK-293) stably transfected with human hERG cDNA were plated on collagen-coated 35-mm cell culture dishes at low density for 1-7 days. For single-cell patch-clamp recording, the intracellular solution contained 130 mM KCl, 10 mM NaCl, 1 mM MgCl₂, 10 mM EGTA, 5 mM MgATP, and 10 mM HEPES (pH adjusted to 7.2 with KOH). The extracellular solution contained acellular Solution (mM): 137 mM NaCl, 4 mM KCl, 1.8 mM CaCl₂, 1 mM MgCl₂, 10 mM D(+)-Glucose, and 10 mM HEPES (pH adjusted to 7.4 with NaOH). All measurements were performed at room temperature.

HCQ, S-HCQ, R-HCQ were added to the extracellular solution at concentrations of 0.3 µM, 1 µM, 3 µM, 10 µM, and 30 µM. Also tested at these concentrations was chloroquine diphosphate (CQ), known to have more serious side-effects than HCQ. Cisapride, a confirmed inhibitor of hERG, was used as a positive control.

After cell capture, a pulse protocol was implemented that involved stepping from a holding potential of -80 mV to +40 mV for 4s to inactivate hERG channels. The membrane voltage was then stepped back to -50 mV for 4s to evoke a tail current prior to returning to the holding potential. This sequence was repeated with an inter-pulse interval of 20s. The voltage protocol was applied throughout the experiment starting prior to compound addition (negative control) and after addition of test compounds, i.e., HCQ, S-HCQ, R-HCQ, CQ, or cisapride (positive control). Evoked peak tail current amplitudes were continuously monitored throughout the experiment. Each compound was applied from low to high concentrations sequentially to the same cell for five minutes.

The percent inhibition of hERG channel was calculated by comparing the tail current amplitude before and after application of the compound, the current difference being normalized to control. The amplitude of the hERG peak tail current was calculated by measuring the tail current on stepping to -50mV. The current was measured before and after each compound addition. Individual cell results were normalized to their respective vehicle control and the results were averaged. Percent inhibition values were used to calculate the IC₅₀ for each tested compound. The higher the IC₅₀ value of a compound in this assay, the less effective it is as an inhibitor of hERG and thus the lower its expected cardiotoxicity.

The results showed that the IC₅₀ for HCQ, R-HCQ, and CQ was 4.84 µM, 4.20 µM, and 0.98 µM, respectively. CQ was a more effective inhibitor of hERG function than HCQ and R-HCQ, confirming the ability of the *in vitro* cardiotoxicity assay to predict *in vivo* side-effects.

Unexpectedly, the IC₅₀ for S-HCQ was 10.96 µM. Having the highest IC₅₀ value, S-HCQ is the least effective inhibitor of hERG tested. As such, S-HCQ would be expected to have the least cardiac toxicity *in vivo* among the tested compounds.

To summarize the above examples, there were no striking differences between S-HCQ and R-HCQ revealed by the *in vitro* pharmacodynamic study (Example 1).

By contrast, the *in vivo* pharmacodynamic study in MRL/lpr mice (Example 2) showed that S-HCQ treatment resulted in a higher fractional survival and lower incidence of lupus-like skin lesions. Further, BUN and creatinine concentrations in plasma were reduced in MRL/lpr mice most significantly in animals treated with 40 mg/kg of S-HCQ, showing that this compound is more protective of kidney function. These results provide a strong rationale to favor S-HCQ over HCQ and R-HCQ for treating lupus patients, particularly those patients with concurrent lupus nephritis.

Finally, S-HCQ was the least cardiotoxic of the tested drugs (Example 3), reinforcing the rationale mentioned above for selecting S-HCQ over HCQ and R-HCQ for treating lupus patients.

The following references, some cited *supra,* can be used to better understand the background of the application:
Chhonker et al. 2018, J Chromatogr B Analyt Technol Biomed Life Sci. 1072:320-327.
Furukawa et al. 1996, J Invest Dermatol. 107(1):95-100.
Furukawa et al. 2009, Autoimmun Rev. 8(6):495-9.
Jiang et al. 2007, J Immunol. 178(11):7422-31.
Keil et al. 2016, Arthritis Res Ther. 18(1):243.
Knight et al. 2015, Ann Rheum Dis. 74(12):2199-206.
Rupanagudi et al. 2015, Ann Rheum Dis. 74(2):452-63.
Shimomatsu et al. 2016, Mod Rheumatol. 26(5):744-8.
Vugmeyster et al. 2010, MAbs. 2(3):335-46.

## Claims

1. S-hydroxychloroquine for use in treating lupus erythematosus in a subject.

2. A pharmaceutical composition for use in treating lupus erythematosus in a subject, wherein the pharmaceutical composition contains S-hydroxychloroquine and a pharmaceutically acceptable excipient, and wherein the pharmaceutical composition is essentially free of R-hydroxychloroquine.

3. The S-hydroxychloroquine for use of claim 1 or the pharmaceutical composition for use of claim 2, wherein the S-hydroxychloroquine is in the form of a pharmaceutically acceptable salt.

4. The S-hydroxychloroquine or the pharmaceutical composition for use of claim 3, wherein the pharmaceutically acceptable salt is a hydrochloride salt, a sulfate salt or a phosphate salt.

5. The S-hydroxychloroquine or the pharmaceutical composition for use of any of claims 1 to 4, wherein the subject is to be administered with a dose of the S-hydroxychloroquine from 100 mg to 800 mg per day.

6. The S-hydroxychloroquine or the pharmaceutical composition for use of claim 5, wherein the dose of the S-hydroxychloroquine is 200 mg per day.

7. The pharmaceutical composition for use of any of claims 2 to 6, wherein the pharmaceutical composition is in the form of granules, a tablet, a capsule, a pill, a powder, a solution, a suspension, or a syrup.

8. The S-hydroxychloroquine or the pharmaceutical composition for use of any of claims 1 to 7, wherein the lupus erythematosus is systemic lupus erythematosus.

9. The S-hydroxychloroquine or the pharmaceutical composition for use of any of claims 1 to 8, wherein the subject suffers from lupus nephritis.

10. The S-hydroxychloroquine or the pharmaceutical composition for use of any of claims 1 to 7, wherein the lupus erythematosus is cutaneous lupus erythematosus.
